# DEMANDE DE BREVET EUROPEEN

(11) **EP 1 563 826 A1**
(43) Date de publication de la demande: **17.08.2005**
(21) Numéro de dépôt: 05290114.7
(22) Date de dépôt: 19.01.2005
(51) Int. Cl.: A61K 7/06, A61K 7/48

(54) **Procédé de préparation d'une compositon de traitement cosmétique à partir de fluide sous pression, et d'acides aminés, de peptides et de protèines.**

(30) Priorité: 29.01.2004 FR 0400846
(71) Demandeur: L'OREAL, 75008 Paris (FR)
(72) Inventeur: De la Mettrie Roland, 78110 Le Vesinet (FR)
(74) Mandataire: Casalonga, Axel

(57) **Abrégé**

La présente invention concerne un procédé de préparation d'une composition de traitement cosmétique des matières kératiniques, par percolation de fluide comprenant au moins de la vapeur d'eau, au travers d'au moins agent de conditionnement choisi parmi les acides aminés, les oligopeptides, les peptides, et les protéines hydrolysées ou non, modifiées ou non, sous forme solide ou pâteuse.

L'invention concerne aussi un dispositif de conditionnement d'un tel agent ainsi que le procédé de traitement cosmétique mettant en oeuvre une telle composition.

## Description

La présente invention a pour objet un procédé de préparation d'une composition cosmétique destinée au traitement des matières kératiniques, en particulier de la peau et des fibres kératiniques humaines telles que les cheveux, ainsi qu'un procédé de traitement cosmétique des matières kératiniques à partir de cette composition.

En cosmétique on cherche toujours à améliorer les propriétés cosmétiques des matières kératiniques, par exemple des cheveux sensibilisés, i.e. qui sont abîmés ou fragilisés sous l'action chimique des agents atmosphériques tels que les radicaux libres, la lumière et la pollution, et/ou des traitements capillaires tels que permanentes, teintures ou décolorations. On peut alors leur appliquer des compositions de traitement cosmétique comprenant des agents de conditionnement tels que des acides aminés, les oligopeptides, les peptides, et les protéines hydrolysées ou non, modifiées ou non, destinés à réparer ou limiter les effets néfastes ou indésirables produits par les différents traitements ou agressions que subissent, de manière plus ou moins répétée, les matières kératiniques. Ces agents de conditionnement peuvent également améliorer les propriétés cosmétiques des cheveux naturels.

Les agents de conditionnement des matières kératiniques, tels que les acides aminés, les oligopeptides, les peptides, et les protéines hydrolysées ou non, modifiées ou non, confèrent notamment, aux cheveux secs ou mouillés, une protection des cheveux contre les agressions extérieures, un renforcement des fibres capillaires et une corporisation.

Ces agents de conditionnement confèrent également à la peau un soin anti-age et permettent une protection du collagène ainsi qu'une nutrition cellulaire.

Cependant, les compositions de traitement cosmétique contenant de tels agents de conditionnement sont généralement des compositions aqueuses dans lesquelles lesdits agents doivent être solubilisés. Le manque de solubilité de ces composés diminue le pouvoir conditionnant de ces compositions. En outre, ce critère de solubilité réduit le nombre d'acides aminés que l'on peut utiliser pour le traitement cosmétique des matières kératiniques. Ceci est particulièrement le cas des composés à point de fusion élevé.

La Demanderesse a découvert de manière surprenante qu'en utilisant un nouveau procédé de préparation d'une composition de traitement cosmétique des matières kératiniques, on pouvait obtenir en un temps très court, inférieur à 2 minutes, des compositions plus ou moins concentrées en agent(s) de conditionnement selon le besoin, notamment sans conservateur, permettant de surmonter les problèmes de solubilité exposés ci-dessus.

Ce procédé est mis en oeuvre simplement et est approprié au besoin du consommateur. On fait passer un fluide, dont la température est de préférence supérieure ou égale à 30 °C, sous pression, pendant un laps de temps très court, inférieur à 1 minute, à travers au moins un agent de conditionnement sous forme solide ou pâteuse, de préférence solide, et encore plus préférentiellement pulvérulente.

Il permet aussi d'utiliser sous forme anhydre des agents de conditionnement instables dans des compositions aqueuses soit parce qu'ils réagissent avec l'eau, soit parce qu'ils réagissent en solution aqueuse avec des composés qui ne réagissent pas avec eux dans une composition anhydre.

Les compositions préparées selon ce procédé peuvent présenter une stabilité au stockage limitée, ce qui n'est pas ici un inconvénient puisque le procédé conduit à une composition prête à l'emploi, destinée à être utilisée rapidement après sa préparation, par exemple dans les 5 minutes suivant sa préparation, notamment après refroidissement jusqu'à une température acceptable pour les matières kératiniques, de préférence inférieure à 60 °C, mieux inférieure à 50 °C. La composition peut être également utilisée jusqu'à une semaine après sa préparation, selon la vitesse de dégradation de l'agent de conditionnement utilisé.

Etant donné le temps de préparation très court, les compositions de traitement cosmétique peuvent être préparées "à la demande" en mélangeant différents composés actifs en cosmétique selon les propriétés cosmétiques recherchées.

Selon un autre mode de réalisation, les agents conditionnement pouvant être conditionnés dans un dispositif prêt-à-l'emploi, il n'est pas nécessaire de déterminer au préalable les concentrations desdits agents en solution, ce qui limite les erreurs de mesure de l'utilisateur.

En outre, le procédé selon l'invention permet d'éviter l'utilisation de flacons multi-compartiments, ce qui rend le procédé particulièrement économique et plus sûr pour l'utilisateur.

La composition ainsi obtenue peut être utilisée seule ou en mélange avec une autre composition.

Un avantage supplémentaire de ce procédé de préparation est l'obtention de compositions présentant de meilleures propriétés cosmétiques. En particulier, les fibres kératiniques traitées avec une composition obtenue par le procédé selon l'invention présentent des propriétés de conditionnement améliorées, et notamment une meilleure protection des cheveux contre les agressions extérieures, un meilleur renforcement des fibres capillaires et une meilleure corporisation, et la peau ainsi traitée est ainsi mieux protégée contre le vieillissement et mieux nourrie.

L'invention a donc pour objet un procédé de préparation d'une composition de traitement cosmétique des matières kératiniques comprenant une étape de percolation d'un fluide sous une pression d'au moins 3 bars au travers d'au moins l'un des acide aminé, oligopeptide, peptide, et protéine hydrolysée ou non, modifiée ou non, sous forme solide ou pâteuse.

Un autre objet de l'invention est une composition susceptible d'être obtenue par le procédé selon l'invention.

L'invention a également pour objet l'utilisation de la composition obtenue selon le procédé de l'invention, pour le traitement cosmétique des matières kératiniques, et notamment le conditionnement des cheveux et de la peau.

L'invention a encore pour objet un dispositif de conditionnement permettant de mettre en oeuvre le procédé de préparation de la présente invention.

D'autres objets, caractéristiques, aspects et avantages de l'invention apparaîtront encore plus clairement à la lecture de la description et des exemples qui suivent.

Selon l'invention, le procédé de préparation d'une composition de traitement cosmétique des matières kératiniques comprend une étape de percolation de fluide, à une température de préférence supérieure ou égale à 30 °C, mieux encore allant de 30 à 150 °C, et encore plus préférentiellement de 40 à 120 °C, sous une pression d'au moins 3 bars (3.10⁵ Pa) au travers au moins l'un des acide aminé, oligopeptide, peptide, et protéine hydrolysée ou non, modifiée ou non, sous forme solide ou pâteuse.

La percolation est un mouvement de fluide à travers un milieu poreux permettant le passage du fluide, sous l'action ou l'effet de la pression.

Le fluide comprend au moins de la vapeur d'eau. Il peut être constitué par de la vapeur d'eau éventuellement accompagnée d'eau liquide, ou par un mélange de vapeur d'eau éventuellement accompagnée d'eau liquide, et d'un ou plusieurs solvants liquides et/ou gazeux cosmétiquement acceptables. De préférence, il est de la vapeur d'eau pouvant être accompagnée d'eau liquide.

A titre de solvant organique, on peut citer, par exemple, les alcools inférieurs en C₁-C₄ tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les acides aminés, oligopeptides, peptides, et protéines hydrolysées ou non, modifiées ou non, utilisés dans l'invention sont sous forme solide ou pâteuse, de préférence sous forme solide, et encore plus préférentiellement pulvérulente.

Par «forme pâteuse» au sens de la présente invention, on entend une consistance intermédiaire entre une phase solide et une phase liquide. La viscosité de cette phase pâteuse est de préférence supérieure à 0,1 Pa.s, et encore plus préférentiellement supérieure à 1 Pa.s, ceci à 25°C avec un taux de cisaillement de 10 s⁻¹.

Par "matières kératiniques", on entend la peau, les lèvres, et/ou les phanères tels que les ongles et les fibres kératiniques, par exemple les cils, les sourcils et les cheveux.

Le procédé de la présente invention peut être mis en oeuvre à partir d'un dispositif classique permettant de générer un fluide sous pression, à une température de préférence supérieure ou égale à 30 °C, mieux encore allant de 30 à 150 °C, et encore plus préférentiellement de 40 à 120 °C. Un tel dispositif comprend une chambre résistant à la pression, équipée d'un bloc thermique, ainsi qu'un circuit d'acheminement du fluide vers l'acide aminé, l'oligopeptide, le peptide, et/ou la protéine hydrolysée ou non, modifiée ou non, utilisés dans l'invention.

Selon un autre mode de réalisation, le dispositif comprend de plus un réservoir de liquide(s) ainsi qu'une pompe permettant l'acheminement du ou des liquides vers la chambre.

Le liquide contenu dans le réservoir est soit de l'eau, soit un mélange d'eau et d'un ou plusieurs solvants cosmétiquement acceptables. De préférence, le liquide est de l'eau.

Un dispositif particulièrement utile pour la mise en oeuvre du procédé de la présente invention est une machine à café du type "expresso". De telles machines sont bien connues de la technique. Par exemple, ces machines sont décrites dans les brevets AT 168405, US 2688911, DE 32433870 et IT 1265636.

Selon un mode de réalisation particulier de l'invention, l'étape de percolation est mise en oeuvre avec un fluide à une température supérieure ou égale à 30 °C, mieux encore allant de 30 à 150 °C, et encore plus préférentiellement de 40 à 120 °C, sous une pression allant de 3 à 30 bar (3.10⁵ à 3.10⁶ Pa), de préférence d'au moins 4 bars (4.10⁵ Pa), mieux encore supérieure ou égale à 10 bars (10⁶ Pa), et tout particulièrement allant de 10 à 30 bars (10⁶ à 3.10⁶ Pa).

Une composition cosmétique comprenant au moins un acide aminé, oligopeptide, peptide, et protéine hydrolysée ou non, modifiée ou non, peut être utilisée directement dans le dispositif générant le fluide sous pression dans un récipient destiné à cet usage. Elle peut aussi être conditionnée dans un dispositif de conditionnement particulier comprenant un logement fermé délimité par au moins une paroi au moins en partie perméable au fluide sous une pression d'au moins 3 bars. De tels dispositifs sont, par exemple, décrits dans les demandes de brevets WO 00/56629, EP512470, US 5897899 ou WO 99/03573. Ces dispositifs de conditionnement sont en général étanches à l'air, l'humidité et/ou la lumière.

Selon un mode de réalisation particulier, le logement est délimité par deux feuilles scellées. Selon un autre mode de réalisation, le logement est délimité par une barquette fermée par un couvercle.

Ces dispositifs peuvent être fabriqués à partir de matériaux tissés ou non tissés, en matière plastique ou végétale, par exemple en cellulose, en métal tel que l'aluminium ou en composite. De tels dispositifs sont par exemple décrits dans les demandes de brevets WO 00/56629, EP 512470 ou WO 99/03573.

Les acides aminés utilisables selon l'invention peuvent être naturels ou synthétiques, et comportent au moins une fonction acide. La ou les fonctions acides peuvent être carboxyliques, sulfoniques, phosphoniques ou phosphoriques.

A titre d'acides aminés utilisables dans la présente invention, on peut notamment citer l'acide aspartique, l'acide glutamique, l'alanine, l'arginine, l'asparagine, la carnitine, la cystéine, la glutamine, la glycine, l'histidine, l'isoleucine, la leucine, la méthionine, la N-phénylalanine, la proline, la serine, la taurine la thréonine, le tryptophane, la tyrosine et la valine.

On peut aussi utiliser des polyaminoacides tels que la polylysine, la polyarginine et l'acide polyaspartique.

Les acides aminés particulièrement préférés dans la présente invention sont l'arginine, la cystéine, la glycine et la taurine.

Comme exemples d'oligopeptide, de peptide, et de protéine hydrolysée ou non, modifiée ou non, utilisés dans l'invention, on peut notamment citer :
- les oligopeptides de synthèse tels que TYR-GLY-GLY-PHE-LEU , triacétate de H-LYS-THR-THR-DAP-SER-OH, HIS-SER-GLN-GLY-THR-PHE, le palmitoyl-TYR-GLY-GLY-PHE-LEU et le palmitoyl-GLY-GLN-PRO-ARG ;
- les protéines végétales issues de végétaux à l'état natif ou présentes dans des farines.
   Parmi les protéines natives, on peut citer les protéines de fèves de soja, les protéines d'avoine, les protéines de pois et en particulier l'albumine de pois, les protéines d'amande, d'abricot, la gliadine de blé, les protéines de froment, l'extensine et la thaumatine.
   Parmi les farines, on peut citer la farine de grain de germe de blé, la farine d'avoine, la farine de soja, la farine de lupin blanc, la farine de blé complet, la farine de riz, la farine de camélia, la farine d'orge, la farine de seigle, la farine de châtaigne, la farine de kinoa et la farine de maïs ;
- les protéines animales produites par les espèces du règne animal, telles que les protéines de lactosérum, la caséine, les protéines de soie (dont la fibroïne), la transglutine, les lactalbumines, les lactoglobulines, les lactoferrines, les protéines de blanc d'oeuf dont l'albumine, les kératines dont celles issues de plumes, le collagène et la gélatine ;
- les hydrolysats de protéines obtenus par hydrolyse chimique ou enzymatique, tels que les peptides ou hydrolysats de protéines de soja, de blé, de maïs, de pommes de terre et de pois ;
- les protéines ou hydrolysats de protéines chimiquement modifiés tels que les esters éthyliques ou propyliques de peptides de blé, le palmitoyl stéaroyl d'alpha lactalbumine, et les hydrolysats de protéines quaternisées.

Le ou les acides aminés, oligopeptides, peptides, et protéines hydrolysées ou non, modifiées ou non, peuvent être mis en oeuvre en mélange avec un ou plusieurs adjuvants solides ou pâteux, et de préférence pulvérulents. Les adjuvants peuvent être choisis parmi les argiles, les sels, les tensioactifs anioniques, non ioniques, cationiques ou zwittérioniques, les épaississants naturels ou de synthèse, l'amidon éventuellement modifié, les billes de verre, la silice, le nylon, l'alumine, le dioxyde de titane, les zéolithes, le poly(méthacrylate de méthyle) (PMMA), le chitosane, la maltodextrine, la cyclodextrine, les mono- ou disaccharides comme le glucose, le saccharose, le sorbitol ou le fructose, l'oxyde de zinc, de zirconium, les silicabades, le talc, les borosilicates notamment de calcium, le polyéthylène, le polytétrafluoroéthylène (PTFE), la cellulose et ses dérivés, les composés superabsorbants, les carbonates de magnésium ou de calcium, le polyacrylamide, l'hydroxyapatite poreux, la sciure de bois, la poudre de fucus, la polyvinylpyrrolidone réticulée, l'alginate de calcium, le charbon actif, les particules de poly(chlorure de vinylidène/acrylonitrile), notamment celles commercialisées sous la dénomination générale d'« Expancel® » par la société AKZO NOBEL sous les références particulières « Expancel® WE» ou « DE » Expancels, et leurs mélanges.

Lorsqu'un ou plusieurs adjuvants sont présents, ledit ou lesdits acides aminés, oligopeptides, peptides, et protéines utilisés dans l'invention sont présents de préférence en une quantité allant de 0,5 à 99% en poids, mieux encore de 1 à 80 % en poids, et encore plus préférentiellement de 2 à 60 % en poids par rapport au poids total acide(s) aminé(s), oligopeptide(s), peptide(s) et/ou protéine(s), et adjuvant(s) sous forme solide ou pâteuse.

Lorsque des plantes ou extraits de plantes sont utilisés dans le procédé selon l'invention, ils peuvent être soumis, avant la percolation, à un traitement tel qu'une torréfaction, un cryobroyage, ou une lyophilisation.

La composition de traitement cosmétique des matières kératiniques obtenue selon le procédé de l'invention contient outre le(s) acide(s) aminé(s), oligopeptide(s), peptide(s) et/ou protéine(s), et le(s) composant(s) du fluide, à savoir l'eau et/ou le(s) solvant(s) cosmétiquement acceptable(s), éventuellement tout ou partie du ou des adjuvants présents dans le mélange sous forme solide ou pâteuse.

L'invention concerne également une composition susceptible d'être obtenue par le procédé selon l'invention, la composition particulièrement préférée ne comprenant aucun agent conservateur.

A partir du procédé de préparation de l'invention, on obtient une composition de traitement cosmétique des matières kératiniques qui peut être appliquée directement sur les matières kératiniques, ou qui peut être mélangée à un milieu cosmétiquement acceptable, ou encore au moins un additif classiquement utilisé en cosmétique pourra y être ajouté par un opérateur. On peut également mélanger au moins deux compositions obtenues par le procédé de l'invention. La composition de traitement cosmétique des matières kératiniques résultant éventuellement de mélange(s) et/ou addition(s) indiqués ci-dessus, sera dénommée ci-après composition finale de traitement cosmétique ou composition finale.

Un mode de réalisation particulier de l'invention consiste à appliquer la composition obtenue par l'intermédiaire d'un dispositif ne nécessitant pas d'intervention humaine et équipé éventuellement d'un moyen de refroidissement.

Un autre mode de réalisation particulier consiste à ingérer la composition de traitement cosmétique obtenue selon le procédé de l'invention lorsque aucun problème de toxicité n'est connue dans le technique, par exemple lorsque l'agent de conditionnement est la taurine.

La quantité desdits acides aminés, oligopeptides, peptides et/ou protéines, présents dans la composition finale de traitement cosmétique est en général comprise entre 0,001 à 50% en poids environ, de préférence entre 0,005 et 30% en poids, et encore plus préférentiellement entre 0,01 et 20% en poids du poids total de la composition finale de traitement cosmétique.

Lorsque la composition de traitement cosmétique obtenue par le procédé de la présente invention est mélangée à un milieu cosmétiquement acceptable, un tel milieu est généralement constitué par de l'eau ou par un mélange d'eau et d'au moins un solvant organique pour solubiliser les composés qui ne seraient pas suffisamment solubles dans l'eau.

Par "cosmétiquement acceptable", on entend un milieu compatible avec les matières kératiniques et notamment la peau, les lèvres et/ou les phanères, et qui en outre, présente un aspect, un toucher, une odeur et éventuellement un goût agréable pour l'utilisateur.

A titre de solvant organique, on peut citer, par exemple, les alcools inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ, et encore plus préférentiellement entre 5 et 30 % en poids environ par rapport au poids total de la composition finale de traitement cosmétique.

Au moins un additif classiquement utilisé en cosmétique peut être également ajouté dans les compositions de traitement cosmétique obtenues selon le procédé de la présente invention. A titre d'exemples de tels additifs, on peut citer des agents tensioactifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement différents de ceux décrits ci-dessus tels que, par exemple, des huiles de silicone, des agents filmogènes, des agents conservateurs, des agents opacifiants.

Les additifs ci-dessus sont en général présents en une quantité comprise pour chacun d'eux entre 0,01 et 20 % en poids par rapport au poids de la composition finale.

Bien entendu, l'homme de l'art veillera à choisir ce ou ces éventuels composés complémentaires de manière telle que les propriétés avantageuses attachées intrinsèquement à la composition de traitement cosmétique conforme à l'invention ne soient pas, ou substantiellement pas, altérées par la ou les adjonctions envisagées.

Le pH de la composition finale de traitement cosmétique est généralement compris entre 3 et 12, et de préférence entre 5 et 11. Il peut être ajusté à la valeur désirée au moyen d'agents acidifiants ou alcalinisants habituellement utilisés en cosmétique ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants, on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule suivante : 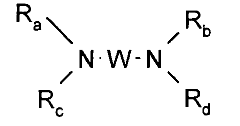 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition finale de traitement cosmétique peut se présenter sous des formes diverses, telles que sous forme de liquides, de crèmes, de gels ou sous toute autre forme appropriée pour réaliser un traitement des matières kératiniques, et notamment de la peau ou des cheveux, et plus particulièrement des cheveux.

La composition finale de traitement cosmétique peut être utilisée, par exemple, comme shampoing, après-shampoing, soin rincé ou non rincé, masque de soin profond, gel douche, lotion ou crème de traitement des matières kératiniques.

La présente invention concerne aussi un procédé de traitement cosmétique des matières kératiniques, comprenant la préparation d'une composition de traitement cosmétique selon le procédé tel que défini ci-dessus, et son application sur les matières kératiniques, par exemple par l'intermédiaire d'un opérateur ou par l'intermédiaire d'un dispositif ne nécessitant pas d'intervention humaine. La durée d'application peut varier entre 15 secondes et 1 heure.

Avant application, la composition de traitement cosmétique obtenue selon le procédé de l'invention, peut être mélangée à un milieu cosmétiquement acceptable et/ou à un ou plusieurs additifs classiquement utilisés en cosmétique tels que décrits ci-dessus.

Un autre mode de réalisation consiste à préparer au moins deux compositions de traitement cosmétique selon le procédé de l'invention, à les mélanger, et à ajouter éventuellement un milieu cosmétiquement acceptable et/ou un ou plusieurs additifs classiquement utilisés en cosmétique tels que décrits ci-dessus, puis à appliquer la composition finale obtenue sur les matières kératiniques.

Les exemples ci-après illustrent la présente invention.

### EXEMPLES

### Exemple 1

On mélange les ingrédients solides suivants dans les proportions indiquées en % en poids par rapport au poids total de mélange solide :
- Taurine (acide 2-aminoéthylsulfonique) 20 %
- Sulfosuccinate de di(2-éthylhexyle) sous forme de sel monosodique vendu sous la dénomination commerciale AEROSOL OT 100 % par la société CYTEC 40 %
- Hydroxypropylguar vendu sous la dénomination commerciale JAGUAR HP 8 par la société RHODIA 40 %

On place 5 g. de ce mélange dans une machine expresso du commerce. On fait ensuite passer de la vapeur d'eau jusqu'à l'obtention d'une composition (A) présentant un volume final de 50 ml.

On obtient ainsi une composition de traitement cosmétique prête à être appliquée sur les cheveux. Les cheveux traités avec cette composition présentent plus de corps.

On peut ajouter deux parties en poids de composition (A), à une partie en poids d'une composition aqueuse (B) contenant 1 % en poids d'hydroxyéthylcellulose pour faciliter l'application.

### Exemple 2

On mélange les ingrédients solides suivants dans les proportions indiquées en % en poids par rapport au poids total de mélange solide :
- L-Glycine 20 %
- Laurylsulfate de sodium en poudre vendu sous la dénomination commerciale Texapon® Z 95 P par la société COGNIS 40 %
- Copolymère acide acrylique/méthacrylate d'alkyle en C₁₀-C₃₀ réticulé vendu sous la dénomination commerciale Carbopol ETD 2020 par la société NOVEON 40 %

On place 5 g. de ce mélange dans une machine expresso du commerce. On fait ensuite passer de la vapeur d'eau jusqu'à l'obtention d'une composition (A) présentant un volume final de 50 ml.

On obtient ainsi une composition de traitement cosmétique prête à être appliquée sur les cheveux. Les cheveux traités avec cette composition présentent plus de corps.

On peut ajouter deux parties en poids de composition (A), à une partie en poids d'une composition aqueuse (B) contenant 1 % en poids d'hydroxyéthylcellulose pour faciliter l'application.

## Revendications

1. Procédé de préparation d'une composition de traitement cosmétique des matières kératiniques, **caractérisé en ce qu'**il comprend une étape de percolation de fluide comprenant au moins de la vapeur d'eau, sous une pression d'au moins 3 bars au travers d'au moins l'un des acide aminé, oligopeptide, peptide, et protéine hydrolysée ou non, modifiée ou non, sous forme solide ou pâteuse.

2. Procédé selon la revendication 1, **caractérisé en ce que** le fluide est constitué par de la vapeur d'eau éventuellement accompagnée d'eau liquide, ou par un mélange de vapeur d'eau éventuellement accompagnée d'eau liquide, et d'un ou plusieurs solvants liquides et/ou gazeux cosmétiquement acceptables.

3. Procédé selon la revendication 1 ou 2, **caractérisé en ce que** l'acide aminé est naturel ou synthétique.

4. Procédé selon l'une quelconque des revendications précédentes, **caractérisé en ce que** la ou les fonctions acides de l'acide aminé sont carboxyliques, sulfoniques, phosphoniques ou phosphoriques.

5. Procédé selon l'une quelconque des revendications précédentes, **caractérisé en ce que** l'acide aminé est choisi parmi l'acide aspartique, l'acide glutamique, l'alanine, l'arginine, l'asparagine, la carnitine, la cystéine, la glutamine, la glycine, l'histidine, l'isoleucine, la leucine, la méthionine, la N-phénylalanine, la proline, la serine, la taurine la thréonine, le tryptophane, la tyrosine, la valine, la polylysine, la polyarginine et l'acide polyaspartique.

6. Procédé selon la revendication 5, **caractérisé en ce que** l'acide aminé est choisi parmi l'arginine, la cystéine, la glycine et la taurine.

7. Procédé selon l'une quelconque des revendications précédentes, **caractérisé en ce que** l'oligopeptide, le peptide et la protéine hydrolysée ou non, modifiée ou non sont choisis parmi TYR-GLY-GLY-PHE-LEU , triacétate de H-LYS-THR-THR-DAP-SER-OH, HIS-SER-GLN-GLY-THR-PHE, le palmitoyl-TYR-GLY-GLY-PHE-LEU et le palmitoyl-GLY-GLN-PRO-ARG ; les protéines végétales issues de végétaux à l'état natif ou présentes dans des farines ; les protéines animales produites par les espèces du règne animal ; les hydrolysats de protéines obtenus par hydrolyse chimique ou enzymatique ; et les protéines ou hydrolysats de protéines chimiquement modifiés.

8. Procédé selon la revendication 7, **caractérisé en ce que** l'oligopeptide, le peptide et la protéine sont choisis parmi les protéines de fèves de soja, les protéines d'avoine, les protéines de pois, les protéines d'amande, d'abricot, la gliadine de blé, les protéines de froment, l'extensine, la thaumatine ; la farine de grain de germe de blé, la farine d'avoine, la farine de soja, la farine de lupin blanc, la farine de blé complet, la farine de riz, la farine de camélia, la farine d'orge, la farine de seigle, la farine de châtaigne, la farine de kinoa, la farine de maïs ; les protéines de lactosérum, la caséine, les protéines de soie, la transglutine, les lactalbumines, les lactoglobulines, les lactoferrines, les protéines de blanc d'oeuf dont l'albumine, les kératines dont celles issues de plumes, le collagène et la gélatine ; les peptides ou hydrolysats de protéines de soja, de blé, de maïs, de pommes de terre, de pois ; les esters éthyliques ou propyliques de peptides de blé, le palmitoyl stéaroyl d'alpha lactalbumine, et les hydrolysats de protéines quaternisées.

9. Procédé selon l'une quelconque des revendications précédentes, **caractérisé en ce que** l'acide aminé, l'oligopeptide, le peptide et la protéine sous forme solide ou pâteuse sont mis en oeuvre en mélange avec au moins un adjuvant.

10. Procédé selon la revendication 9, **caractérisé en ce que** l'adjuvant est choisi parmi les argiles, les sels, les tensioactifs anioniques, non ioniques, cationiques ou zwittérioniques, les épaississants naturels ou de synthèse, l'amidon éventuellement modifié, les billes de verre, la silice, le nylon, l'alumine, le dioxyde de titane, les zéolithes, le poly(méthacrylate de méthyle) (PMMA), le chitosane, la maltodextrine, la cyclodextrine, les mono- ou disaccharides, l'oxyde de zinc, de zirconium, les silicabades, le talc, les borosilicates notamment de calcium, le polyéthylène, le polytétrafluoroéthylène (PTFE), la cellulose et ses dérivés, les composés superabsorbants, les carbonates de magnésium ou de calcium, le polyacrylamide, l'hydroxyapatite poreux, la sciure de bois, la poudre de fucus, la polyvinylpyrrolidone réticulée, l'alginate de calcium, le charbon actif, et les particules de poly(chlorure de vinylidène/acrylonitrile).

11. Procédé selon la revendication 9 ou 10, **caractérisé en ce que** les acides aminés, oligopeptides, peptides et/ou protéines sont présents en une quantité de 0,5 à 99% en poids, de préférence de 1 à 80 % en poids par rapport au poids total acide(s) aminé(s), oligopeptide(s), peptide(s) et/ou protéine(s) et adjuvant(s) sous forme solide ou pâteuse.

12. Procédé selon l'une quelconque des revendications 9 à 11, **caractérisé en ce que** la composition de traitement cosmétique obtenue contient outre le(s) acide(s) aminé(s), oligopeptide(s), peptide(s) et/ou protéine(s) et le(s) composant(s) du fluide, éventuellement tout ou partie du ou des adjuvants présents dans le mélange sous forme solide ou pâteuse.

13. Procédé selon l'une quelconque des revendications précédentes, **caractérisé en ce que** l'étape de percolation est mise en oeuvre avec un fluide sous une pression allant de 3 à 30 bar.

14. Procédé, selon la revendication 13, **caractérisé en ce que** l'étape de percolation est mise en oeuvre avec un fluide sous une pression d'au moins 10 bars.

15. Composition de traitement cosmétique des matières kératiniques susceptible d'être obtenue par le procédé selon l'une quelconque des revendications précédentes.

16. Composition de traitement cosmétique des matières kératiniques selon la revendication 15, ne comprenant pas de conservateur.

17. Procédé de traitement cosmétique des matières kératiniques, **caractérisé en ce que** l'on prépare une composition de traitement cosmétique selon le procédé défini selon l'une quelconque des revendications 1 à 14, et que l'on applique cette composition sur les matières kératiniques.

18. Procédé de traitement cosmétique des matières kératiniques selon la revendication 17, **caractérisé en ce que** l'on applique cette composition sur les matières kératiniques par l'intermédiaire d'un dispositif ne nécessitant pas d'intervention humaine.

19. Procédé de traitement cosmétique des matières kératiniques selon la revendication 17, **caractérisé en ce que**, avant l'application, la composition de traitement cosmétique préparée selon le procédé selon l'une quelconque des revendications 1 à 14, est mélangée à un milieu cosmétiquement acceptable et/ou à un ou plusieurs additifs utilisés en cosmétique.

20. Procédé de traitement cosmétique des matières kératiniques, **caractérisé en ce que** l'on prépare au moins deux compositions de traitement cosmétique selon le procédé défini selon l'une quelconque des revendications 1 à 14, on les mélange et on applique le mélange sur les matières kératiniques.

21. Dispositif de conditionnement d'une composition cosmétique, comprenant un logement fermé délimité par au moins une paroi au moins en partie perméable à de la vapeur d'eau sous pression d'au moins 3 bars, ladite composition contenant au moins l'un des acide aminé, oligopeptide, peptide, et protéine hydrolysée ou non, modifiée ou non, sous forme solide ou pâteuse.

22. Dispositif selon la revendication 21, **caractérisé en ce que** le logement est délimité par deux feuilles scellées.

23. Dispositif selon la revendication 21, **caractérisé en ce que** le logement est délimité par une barquette fermée par un couvercle.

24. Utilisation de la composition obtenue par le procédé selon l'une quelconque des revendications 1 à 14, pour le traitement cosmétique des matières kératiniques.

25. Utilisation selon la revendication 24, pour le conditionnement des cheveux ou de la peau.
